# EUROPÄISCHE PATENTSCHRIFT

(11) **EP 2 091 643 B1**
(45) Veröffentlichungstag und Bekanntmachung des Hinweises auf die Patenterteilung: **09.03.2016**
(21) Anmeldenummer: 07822540.6
(22) Anmeldetag: 13.11.2007
(51) Int. Cl.: B01J 13/02

(54) **VERFAHREN ZUR HERSTELLUNG EINER MINISUSPOEMULSION ODER SUSPENSION VON SUBMIKRONEN KERN/SCHALE-PARTIKELN**
METHOD FOR PRODUCTION OF A MINI SUSPOEMULSION OR SUSPENSION OF SUB-MICRON CORE/SHELL PARTICLES
PROCÉDÉ DE PRODUCTION D'UNE MINISUSPOÉMULSION OU D'UNE SUSPENSION DE PARTICULES COEUR-ÉCORCE SUBMICRONIQUES

(30) Priorität: 14.11.2006 EP 06124078; 06.02.2007 EP 07101775
(43) Veröffentlichungstag der Anmeldung: 26.08.2009
(73) Patentinhaber: BASF SE, 67056 Ludwigshafen (DE)
(72) Erfinder: DANNER, Thomas, 67167 Erpolzheim (DE); SACHWEH, Bernd, 67149 Meckenheim (DE); VIERECK, Sonja, 68163 Mannheim (DE)
(86) Internationale Anmeldenummer: PCT/EP2007/062266
(87) Internationale Veröffentlichungsnummer: WO 2008/058958

(56) Entgegenhaltungen:
- EP-A- 0 209 879
- EP-A- 0 542 133
- WO-A-03/006151
- US-A- 4 421 660
- US-A1- 2005 129 946

## Beschreibung

Die Erfindung betrifft ein Verfahren zur Herstellung einer Minisuspoemulsion oder einer Suspension von submikronen Kern/Schale-Partikeln.

Submikrone Kern/Schale-Partikel sind bekannt und bezeichnen Kern/Schale-Partikel mit einem mittleren Außendurchmesser unterhalb von 1 Mikrometer.

Sie werden insbesondere durch Flammsynthese, Fällung, beispielsweise im Zentrifugalfeld oder durch Mahlen hergestellt. Für verschiedene Anwendungen ist es erforderlich, die submikronen Kern/Schale-Partikel in Suspension zu bringen. Hierfür wurden bislang die submikronen Kern/Schale-Partikel zunächst nach einem der oben angegebenen Verfahren hergestellt und im Falle eines pulverförmigen Produktes anschließend unter Zugabe eines flüssigen Mediums in Suspension gebracht. Nachteilig ist, dass die so hergestellten submikronen Kern/Schale-Partikel zu Agglomeration tendieren, schlecht redispergierbar sind und eine mangelnde Langzeitstabilität aufweisen.

US 2005/129946 offenbart ein Verfahren zur Herstellung von Mikrokapseln, dadurch gekennzeichnet, dass von einer Dispersion einer flüssigen organische Phase in einer wässrigen Phase ausgegangen wird, die in der flüssigen organischen Phase Farbpartikel sowie ein Harz enthält, und dass in der flüssigen organische Phase die Mikrokapseln durch Vernetzung des Harzes hergestellt werden. Die Mikrokapseln haben eine mittlere Teilchengröße von 1 bis 1000 µm und werden verwendet zwischen zwei Elektroden für das Anzeigen eines Bildes.

Als Suspensionen werden in bekannter Weise Gemische bezeichnet, enthaltend in einer kontinuierlichen flüssigen Phase eine disperse Feststoffphase.

Als **Emulsionen** werden flüssigdisperse Systeme bezeichnet, die aus zwei nicht miteinander mischbaren flüssigen Phasen bestehen, wovon die eine Phase, die als disperse oder innere Phase bezeichnet wird, in der zweiten, als kontinuierliche oder homogene Phase bezeichneten Phase, in Form feiner Tröpfchen dispergiert vorliegt. Je nach Polarität der Phasen werden Emulsionen als Öl-in-Wasser- (O/W-) bzw. Wasser-in-Öl- (W/O-) Emulsionen bezeichnet, wobei im ersten Fall eine Ölphase, bestehend aus unpolaren Medien, in einer polaren Phase, bestehend aus einer wässrigen Lösung oder anderen, mit der unpolaren Phase nicht mischbaren Verbindungen, in Form fein dispergierter Tropfen vorliegt. Im Fall der W/O-Emulsion liegt umgekehrt die polare Phase in Form fein dispergierter Tropfen in der Ölphase vor. Der Anteil der dispersen Phase an der gesamten Emulsion kann in einem Bereich von > 0 % bis < 100 % liegen.

Der Begriff "Miniemulsion" wird für kinetisch durch sterische und/oder elektrostatische Effekte und/oder durch ein oder mehrere Tenside und/oder durch weitere Hilfsstoffe stabilisierte, thermodynamisch instabile flüssigdisperse Systeme (Emulsionen) verwendet, deren disperse Phase einen mittleren Tröpfchendurchmesser ≦ 5000 nm (≦ 5 µm) aufweist.

Der Begriff Suspoemulsion wird für Mischungen verwendet, die in einer Emulsion verteilte Feststoffpartikel aufweisen und entsprechend als Minisuspoemulsion eine Miniemulsion mit darin verteilten Feststoffpartikeln.

Die Herstellung einer Miniemulsion kann beispielsweise unter Zuführung von mechanischer Energie, beispielsweise in Form von Rührenergie, turbulenter kinetischer Energie, Ultraschallwellen, Druck mit nachfolgender Entspannung über ein Homogenisierventil, mittels statischer Mischer, Mikromischer oder Membranen bzw. allgemein durch Aufprägen von laminaren oder turbulenten Scher- und/oder Dehnströmungen und Kavitation erfolgen. Die entstehende Emulsionsart (W/O-Emulsion oder O/W-Emulsion) wird durch die Auswahl des Stoffsystems der dispersen und kontinuierlichen Phasen, des oder der Tenside und/oder der verwendeten Hilfsstoffe bestimmt.

Voraussetzung bei der Emulgierung ist, dass die Tropfen in der Miniemulsion oder in der Suspoemulsion für die Dauer der Herstellung von Kern/Schale-Partikeln ausreichend stabil sind. Dies kann je nach Stoffsystem durch Oberflächenladung, also elektrostatische Abstoßung, der Tropfen an sich gegeben sein. Ist eine fremde Stabilisierung der Tropfenphase durch Tenside nötig, so kann diese über elektrostatische und/oder sterische Effekte, die durch geeignete Stabilisierungshilfsmittel hervorgerufen werden, oder Pickering-Stabilisatoren (grenzflächenaktive Partikel) erfolgen, die in der flüssigen, kontinuierlichen Phase enthalten sind. Auch in der flüssigen, dispersen Phase können Hilfsmittel zur Stabilisierung der Partikel und/oder der Emulsion enthalten sein. Hilfsmittel zur Stabilisierung der Miniemulsion oder der submikronen Suspension schließen auch Substanzen ein, die die rheologischen Eigenschaften der kontinuierlichen Phase derartig verändern, dass Aufrahmen oder Sedimentation der Tropfen oder der Partikel der dispersen Phase verhindert oder verlangsamt werden.

Als flüssig werden vorliegend Phasen verstanden, die unter den Bedingungen der vorliegend beschriebenen Verfahren im flüssigen Aggregatzustand sind.

Es war Aufgabe der Erfindung, ein Verfahren zur Herstellung einer Minisuspoemulsion oder einer Suspension von submikronen Kern/Schale-Partikeln zur Verfügung zu stellen, wonach die Schale maßgeschneidert, gezielt, insbesondere auf alle Kern partikel in gleicher Weise aufgebracht werden kann, und das es ermöglicht, die gegebene Polydispersität der submikronen Ausgangs-Suspension eines oder mehrerer den Kern der submikronen Kern/Schale-Partikel bildenden Feststoffe im Wesentlichen beizubehalten und submikronen Kern/Schale-Partikel in großtechnischem Maßstab in einer dem Labormaßstab vergleichbaren Qualität zur Verfügung zu stellen. Die Schale der submikrone Kern/Schale-Partikel kann offen oder geschlossen sein, was sich in einem Hüllenschlussindex (belegte Oberfläche/Kernpartikeloberfläche) ausdrückt.

Die Aufgabe wird gelöst durch ein Verfahren zur Herstellung einer Minisuspoemulsion oder einer Suspension von submikronen Kern/Schale-Partikeln, das dadurch gekennzeichnet ist, dass
- von einer Suspoemulsion einer ersten, dispersen flüssigen Phase I, in einer zweiten, kontinuierlichen flüssigen Phase II ausgegangen wird, die
- in der ersten, dispersen flüssigen Phase I submikrone Partikel eines den Kern bildenden Feststoffes K sowie
- eine molekular dispers gelöste Vorläufersubstanz VS für die Schale und gegebenenfalls einen Reaktanden R enthält, und dass
- in der ersten, dispersen flüssigen Phase I der Suspoemulsion die submikronen Kern/Schale-Partikel KS durch chemische oder physikalische Umwandlung der Vorläufersubstanz VS für die Schale hergestellt werden.

Vorliegend werden unter dem Begriff submikrone Partikel eines den Kern bildenden Feststoffes K auch submikrone Partikel eines oder mehrerer den Kern bildenden Feststoffe K verstanden. Analog werden unter einer molekular dispers gelösten Vorläufersubstanz VS für die Schale auch mehrere molekular dispers gelöste Vorläufersubstanzen VS für die Schale, und unter einer molekular dispers gelösten Vorläufersubstanz VK für den Kern auch mehrere molekular dispers gelöste Vorläufersubstanzen VK für den Kern verstanden.

Es wurde gefunden, dass es möglich ist, in den Tropfen einer ersten flüssigen Phase I, die sowohl den einen oder die mehreren den Kern bildenden Feststoffe als auch die eine oder die mehreren molekular dispers gelösten Vorläufersubstanzen des die Schale bildenden Feststoffes enthalten, aus den molekular dispers gelösten Vorläufersubstanzen auf den Feststöffkernen die Schale auszubilden. Dabei fungieren die Tropfen der ersten flüssigen Phase I aufgrund ihrer geringen Abmessungen - es handelt sich um eine Minisuspoemulsion -, und entsprechend, wie eingangs definiert, um mittlere Tröpfchendurchmesser der dispersen Phase ≦ 5 µm, als Minireaktoren, die gewährleisten, dass die gegebene Polydispersität der submikronen Ausgangs-Suspension der den Kern bildenden Feststoffe im Wesentlichen beibehalten wird.

Hierzu wird von einer Suspoemulsion einer ersten, dispersen flüssigen Phase I, in einer zweiten, kontinuierlichen flüssigen Phase II ausgegangen, die in der ersten, dispersen flüssigen Phase I submikrone Partikel eines den Kern bildenden Feststoffes K sowie eine molekular dispers gelöste Vorläufersubstanz VS für die Schale enthält.

In einer Ausführungsform enthält die obige Suspoemulsion zusätzlich einen Reaktanden R.

Die oben beschriebene Suspoemulsion kann in einer ersten Ausführungsform hergestellt werden, indem
- von einer Suspension der submikronen Partikel des den Kern bildenden Feststoffes K in der ersten flüssigen Phase I ausgegangen,
- hierzu die Vorläufersubstanz VS für die Schale zugegeben und molekular dispers gelöst und anschließend
- die zweite flüssige Phase II zugegeben und unter Zuführung von Energie mit der ersten flüssigen Phase I emulgiert wird.

In einer weiteren Ausführungsform wird die obige Suspoemulsion hergestellt, indem
- von einer Suspoemulsion von submikronen Partikeln eines den Kern bildenden Feststoffes K in der ersten flüssigen Phase I als disperser Phase, in der zweiten flüssigen Phase II als kontinuierlicher Phase ausgegangen wird,
- die Vorläufersubstanz VS für die Schale in einer dritten flüssigen Phase III eingebracht wird, die mit der ersten flüssigen Phase I mischbar ist, jedoch mit der zweiten flüssigen Phase II nicht mischbar ist, und
- aus der dritten flüssigen Phase III, enthaltend die Vorläufersubstanz VS, mit einer vierten flüssigen Phase IV, die mit der zweiten flüssigen Phase II mischbar ist, nicht jedoch mit der ersten und der dritten flüssigen Phase III, unter Energieeintrag eine Emulsion gebildet wird, und
- die Tropfen der ersten flüssigen Phase I und die Tropfen der dritten flüssigen Phase III durch Energieeintrag zur Koaleszenz gebracht werden.

In dieser Ausführungsform wird somit die disperse Phase einer ersten Suspoemulsion, enthaltend in den Tropfen der dispersen Phase die submikronen Partikel des den Kern bildenden Feststoffes, mit der dispersen Phase einer zweiten Emulsion, enthaltend in den Tropfen der dispersen Phase die Vorläufersubstanz VS für die Schale, zur erzwungenen Koaleszenz gebracht.

In einer weiteren Ausführungsform wird die obige Suspoemulsion zur Verfügung gestellt, die in den Tropfen der dispersen Phase zusätzlich zu den submikronen Partikeln des den Kern bildenden Feststoffes K und der molekular dispers gelösten Vorläufersubstanz VS für die Schale einen Reaktanden R enthält. Hierzu wird
- von einer Suspoemulsion von submikronen Partikeln eines den Kern bildenden Feststoffes K sowie einer molekular dispers gelösten Vorläufersubstanz VS für die Schale in der ersten flüssigen Phase I als disperser Phase, in der zweiten flüssigen Phase II als kontinuierlicher Phase ausgegangen,
- wonach ein Reaktand R entweder in der zweiten, kontinuierlichen flüssigen Phase II zugegeben wird und in die Tropfen der ersten, dispersen flüssigen Phase I eindiffundiert oder
- in einer weiteren flüssigen Phase V zugegeben wird, die mit der ersten, dispersen flüssigen Phase I mischbar ist, jedoch nicht mit der zweiten, kontinuierlichen flüssigen Phase II
- aus der weiteren flüssigen Phase V, enthaltend den Reaktanden R mit einer zusätzlichen flüssigen Phase VI, unter Energieeintrag eine Emulsion gebildet wird und die Tropfen derselben
- mit den Tropfen der ersten, dispersen flüssigen Phase I, enthaltend den den Kern bildenden Feststoff K sowie die Vorläufersubstanz VS für die Schale, zur erzwungenen Koaleszenz gebracht wird.

In einer weiteren Ausführungsform wird die obige Suspoemulsion, enthaltend zusätzlich zu den submikronen Partikeln des den Kern bildenden Feststoffes K und der molekular dispers gelösten Vorläufersubstanz VS für die Schale den Reaktanden R durch erzwungene Koaleszenz der Tropfen einer Suspoemulsion, enthaltend die submikronen Partikel des Feststoffes K und den Reaktanden R, mit den Tropfen einer weiteren Emulsion, enthaltend in den Tropfen der dispersen Phase die Vorläufersubstanz VS für die Schale, hergestellt.

Die Herstellung der Suspoemulsion, enthaltend die submikronen Partikel des den Kern bildenden Feststoffes K in der ersten, dispersen flüssigen Phase I, mit der zweiten flüssigen Phase II als kontinuierlicher Phase, kann analog zu den oben beschriebenen Herstellungsverfahren für die Suspoemulsion, enthaltend in den Tropfen der dispersen Phase die submikronen den Kern bildenden Feststoffpartikel K, die molekular dispers gelöste Vorläufersubstanz VS für die Schale und ggf. den Reaktanden R, erfolgen. In einer ersten Ausführungsform wird hierzu
- von einer Miniemulsion ausgegangen, enthaltend eine Vorläufersubstanz VK für den den Kern bildenden Feststoff in der ersten, dispersen flüssigen Phase I, wobei die zweite flüssige Phase II die kontinuierliche Phase ist, und woraus durch physikalische oder chemische Umwandlung der Vorläufersubstanz VK des den Kern bildenden Feststoffes die Minisuspoemulsion des den Kern bildenden Feststoffes K in der ersten, dispersen flüssigen Phase I, mit der zweiten flüssigen Phase II als kontinuierlicher Phase, gebildet wird.

In einer weiteren Ausführungsform wird hierzu
- von einer Miniemulsion ausgegangen, enthaltend eine Vorläufersubstanz VK für den den Kern bildenden Feststoff in der ersten, dispersen flüssigen Phase I, wobei die zweite flüssige Phase II die kontinuierliche Phase ist,
- wonach ein Reaktand R entweder der zweiten, kontinuierlichen flüssigen Phase II zugesetzt wird und in die Tropfen der ersten flüssigen Phase I eindiffundiert oder
- in einer dritten flüssigen Phase III zugesetzt wird, die mit der ersten flüssigen Phase I mischbar ist, jedoch mit der zweiten flüssigen Phase II nicht mischbar ist, und
- aus der dritten flüssigen Phase III, enthaltend den Reaktanden R mit einer vierten flüssigen Phase IV, die mit der zweiten flüssigen Phase II mischbar ist, nicht jedoch mit der ersten und der dritten flüssigen Phase, unter Energieeintrag eine Emulsion gebildet und mit den Tropfen der ersten, dispersen flüssigen Phase I, enthaltend die Vorläufersubstanz VK für den den Kern bildenden Feststoff zur erzwungenen Koaleszenz gebracht wird, und wonach
- die Vorläufersubstanz VK für den den Kern bildenden Feststoff K mit dem Reaktanden R zur chemischen Umsetzung gebracht wird.

In der Ausführungsform, in der die submikronen Kern/Schale-Partikel aus der Suspoemulsion, enthaltend in den Tropfen der dispersen Phase die submikronen Partikel des den Kern bildenden Feststoffes K sowie die molekular dispers gelöste Vorläufersubstanz VS für die Schale, durch physikalische Umwandlung der Vorläufersubstanz VS für die Schale hergestellt werden, kann die physikalische Umwandlung insbesondere durch die Veränderung eines oder mehrerer Verfahrensparameter, bevorzugt der Temperatur und/oder des Druckes oder auch durch die Zugabe eines Lösungsmittels oder eines Salzes analog der zuvor beschriebenen Zugabe eines Reaktanden R ausgelöst werden. Die physikalische Umwandlung kann insbesondere durch Kühlung oder durch Verdampfung des Lösungsmittels des oder der gelösten Feststoffe der Schale bzw. des Kerns oder durch Zugabe eines weiteren Lösungsmittels analog der zuvor beschriebenen Zugabe eines Reaktanden R, das die Löslichkeit des oder der gelösten Feststoffe der Schale bzw. des Kerns herabsetzt, oder durch Zugabe eines oder mehrerer weiterer Salze analog der zuvor beschriebenen Zugabe eines Reaktanden R, die die Löslichkeit des oder der gelösten Feststoffe der Schale bzw. des Kerns herabsetzen, stattfinden.

Als submikrone Partikel werden bekannterweise Feststoffpartikel bezeichnet, deren mittlere Außendurchmesser unterhalb von einem Mikrometer liegen. Insbesondere kann es sich dabei um Nanopartikel handeln, deren mittlerer Außendurchmesser im Nanometerbereich, insbesondere unter 100 nm, liegt. Der mittlere Außendurchmesser der Nanopartikel kann bevorzugt ≧ 1 nm und ≦ 1000 nm sein, weiter bevorzugt ≧ 1 nm und ≦ 100 nm, insbesondere ≧ 1 nm und ≦ 20 nm. Die Dicke der Schale der submikronen Kern/Schale-Partikel kann bevorzugt in einem Bereich von ≧ 0,5 nm und ≦ 100 nm, weiter bevorzugt ≧ 0,5 nm und ≦ 30 nm, insbesondere ≧ 0,5 nm und ≦ 10 nm liegen.

Die Partikelgrößenverteilung kann in an sich bekannter Weise, beispielsweise mittels der Methode der statischen Lichtstreuung, dynamischen Lichtstreuung oder der analytischen Ultrazentrifuge (siehe beispielsweise W. Mächtle, Makromolekulare Chemie 185 (1984), Seiten 1025 bis 1039), aber auch anhand elektronenmikroskopischer Aufnahmen bestimmt werden.

Die submikrone Suspension enthält den oder die den Kern bildenden Feststoffe K bevorzugt in einem Anteil von 0,01 bis 40 Gew.-%, insbesondere in einem Anteil von 1 bis 10 Gew.-%, bezogen auf das Gesamtgewicht der submikronen Suspension.

Der den Kern bildende Feststoff K oder der die Schale bildende Feststoff S kann insbesondere eine Substanz oder eine Mischung von Substanzen, ausgewählt aus der nachfolgenden Aufzählung sein: Metalle, Oxide, Halbleiter, Ruß, Metallsalze, Schwefel, Schwefelverbindungen, Siliziumverbindungen, Polymere, anorganische oder organische Pigmente oder feste anorganische oder organische Wirkstoffe für Kosmetika, Pflanzenschutz-, Tierernährungs- oder Nahrungsergänzungsmittel.

Bevorzugt sind hierbei ein oder mehrere Metalle, ausgewählt aus der nachfolgenden Aufzählung: Au, Ag, Ni, Pd, Fe, Sn, Zn, Co, Cu, Bi, Ce, Zr, Ti oder ein oder mehrere Oxide, ausgewählt aus der nachfolgenden Aufzählung: Schichtsilikate, TiO₂, ZnO, SiO₂, Bi₂O₃, Fe₂O₃, CeOₓ, MFeₓO_{y}, wobei M ein Übergangsmetall oder ein Hauptgruppenmetall ist, ZrO₂, SnO, SnO₂, AlₓO_{y}, CuO, Cu₂O, CaCO₃, oder ein oder mehrere Halbleiter, ausgewählt aus Sulfiden und Seleniden, oder Silizumverbindungen, oder Metallsalze, ausgewählt aus Nitraten, Carbiden, Carbonaten, Sulfaten, Halogeniden, Acetaten, Salze von organischen Säuren, wie Lactaten, Carboxylaten, Carbonsäuren, Hydroxysäuren, oder Polymere, ausgewählt aus PET, Polyacrylnitril, Polystyrol, Polyketon, Polycarbonat, PMMA, PU oder Polybutadienterephthalat.

Als molekular disperse Vorläufersubstanz VS des die Schale bildenden Feststoffes S oder als Vorläufersubstanz VK des den Kern bildenden Feststoffes K werden bevorzugt ein oder mehrere organische oder anorganische Salze, insbesondere Zinnsalze, Zinksalze, Cersalze, Eisensalze, Zirkoniumsalze, Bismutsalze oder Kupfersalze oder anorganische Metallverbindungen, insbesondere des Titans, Monomere und/oder ein oder mehrere Siliziumverbindungen eingesetzt.

Beim Reaktand kann es sich beispielsweise um eine in Organik lösliche Base, insbesondere ein Amin, oder eine wasserlösliche Base, wie eine wässrige Natriumhydroxid- oder Kaliumhydroxid-Lösung, oder auch ein Gas wie Kohlendioxid oder Ammoniak, ein Reduktionsmittel (H₂, NaBH₄), ein Oxidationsmittel, Initiatoren, um eine Pufferlösung oder um Ionenaustauscherharze handeln.

Von den beiden nicht miteinander mischbaren Flüssigkeiten ist eine hydrophil und die andere hydrophob.

Bevorzugt ist die erste, disperse flüssige Phase I wässrig und die zweite, kontinuierliche flüssige Phase II eine organische Phase (insbesondere ein Alkan oder eine Mischung von Alkanen, ein pflanzliches Öl oder eine Mischung von pflanzlichen Ölen, ein Silikonöl oder eine Mischung von Silikonölen oder eine Mischung der aufgeführten Substanzen.)

In einer Ausführungsvariante kann aus der Minisuspoemulsion der submikronen Kern/Schale-Partikel durch Abziehen der ersten, dispersen flüssigen Phase I oder der zweiten, kontinuierlichen flüssigen Phase II eine Suspension von submikronen Kern/Schale-Partikeln in der jeweils anderen flüssigen Phase erhalten werden.

In einer vorteilhaften Ausführungsvariante können zwei oder mehrere, insbesondere 2 bis 1000 den Kern bildende Partikel gemeinsam von einer Schale umhüllt werden. Dadurch werden sogenannte Multi-Core-Partikel erhalten. Hierbei muss die Schale nicht vollständig geschlossen sein.

Es ist daher möglich, pro Tropfen der ersten dispersen flüssigen Phase I mehrere Multi-Core-Partikel zu erhalten, je nach Fällbedingungen, was in den Tropfen der ersten dispersen flüssigen Phase mit Abmessungen im Nanometerbereich besser kontrollierbar ist als in der Bulk-Synthese.

In einer weiteren Ausführungsvariante können alle den Kern bildenden Partikel, die in einem Tropfen der ersten dispersen flüssigen Phase I enthalten sind, gemeinsam von einer Schale umhüllt werden. Da alle Tropfen im Wesentlichen gleich groß sind und im Wesentlichen die gleiche Konzentration an Kernpartikeln enthalten, kann in dieser Ausführungsform über die Konzentration der Kernpartikel und die Größe der Tropfen die Größe der erhaltenen Multi-Core-Partikel definiert eingestellt werden, und es werden Multi-Core-Partikel mit sehr einheitlicher Verteilungsbreite erhalten.

Vorteilhaft können auf die den Kern bildenden Partikel zwei bis zehn, bevorzugt zwei bis drei, Schalen übereinander aufgebracht werden.

In einer vorteilhaften Ausführungsform kann der Kern und die eine oder die mehreren Schalen jeweils eine gleiche chemische Zusammensetzung, jedoch eine unterschiedliche Modifikation, insbesondere eine unterschiedliche Kristallstruktur aufweisen, oder es kann das Material des Kerns amorph und das Material der einen oder der mehreren Schalen kristallin sein, oder umgekehrt.

In einer weiteren Ausführungsform wird eine Minisuspoemulsion von submikronen Kern/Schale-Partikeln hergestellt, bei denen die Schale den Kern nicht vollständig umschließt, wonach die submikronen Kern/Schale-Partikel einem nachgeschalteten Verfahrensschritt unterworfen werden, in dem der Kern vollständig oder teilweise, insbesondere durch Verdampfen, Lösen oder Ätzen, unter Erhalt einer Hohlstruktur entfernt wird und nachfolgend auf dieselbe bevorzugt eine weitere Schale oder mehrere weitere Schalen aufgebracht werden können.

Die speziellen Einsatzgebiete der nach dem erfindungsgemäßen Verfahren erhaltenen Kern/Schale-Nanopartikelsuspensionen oder -suspoemulsionen richten sich nach der chemischen Zusammensetzung der submikronen Kern/Schale-Partikel, der verwendeten Stabilisierungshilfsmittel sowie der die flüssige Phase I, die flüssige Phase II bzw. die flüssigen Phasen I und II bildenden Substanzen. Bevorzugte Einsatzgebiete sind als Katalysatoren, als Wirkstoff-Formulierung, insbesondere in Kosmetika, Pflanzenschutz-, Tierernährungs- oder Nahrungsergänzungsmitteln, als Pigmente, in der Elektronik, in optischen Anwendungen oder in Polymeren.

Die Erfindung wird im Folgenden anhand von Ausführungsbeispielen näher erläutert.

### Beispiel 1

Ausgegangen wurde von der Suspension einer ersten, dispersen flüssigen Phase I umfassend mit Aminopropyltrimethoxysilan oberflächenmodifizierte Siliziumdioxidpartikel mit einer mittleren Partikelgröße von 20 nm in Wasser, Quadrol L® der BASF AG, d.h. Tetrahydroxypropylethylendiamin, CAS-Nr. 102-60-3 als Tensid und Zinn(II)chlorid. Die Suspoemulsion umfasste die erste, disperse flüssige Phase I in einer zweiten kontinuierlichen flüssigen Phase aus n-Dekan und Glissopal EM23® der BASF AG, einem Tensid aus einem zwitterionischen Molekül auf der Basis von Polyisobuten mit der mittleren Molmasse von ca. 1000 g/mol.

Die erste disperse flüssige Phase wurde hergestellt, indem 0,75 g des Tensids Quadrol L®, und 2,25 g Zinn(II)chlorid unter Rühren in 96 g Wasser gelöst wurden. Anschließend wurden 0,75 g oberflächenmodifizierte Siliziumdioxidpartikel hinzugegeben.

Die zweite, kontinuierliche flüssige Phase wurde hergestellt, indem 4,5 g Glissopal EM23® unter Rühren in 199 g n-Dekan gelöst wurden.

Die erste disperse flüssige Phase und die zweite kontinuierliche flüssige Phase wurden in einem Ultra-Turrax®-Mischgerät der Firma IKA®-Werke GmbH & Co. KG (Staufen, Deutschland) voremulgiert. Anschließend wurde die so hergestellte Roh-Suspoemulsion durch Anlegen von Vakuum entgast.

Diese Roh-Suspoemulsion wurde in einem Hochdruckhomogenisator emulgiert, so dass sich eine Tropfengrößenverteilung mit Sauterdurchmesser x_{3,2} von 530 nm einstellte. Anschließend wurde diese Feinemulsion durch Anlegen von Vakuum entgast.

In diese Feinemulsion wurden 5,63 g Pyridin hinzugegeben, so dass sich das Pyridin in der kontinuierlichen Phase der Suspoemulsion löste. Anschließend wurde auf 120°C erhitzt und diese Temperatur 4 Stunden lang gehalten.

Auf diese Weise konnten Partikel mit einer Kern-Schale-Struktur hergestellt werden. Zinndioxid lagerte sich als 5 nm-Partikel auf der Oberfläche der Siliziumdioxidkernpartikel ab.

Von der so erhaltenen Minisuspoemulsion von submikronen Kern/Schale-Partikeln wurden alle Flüssigkeiten abgetrennt, um eine High Angle Annular Dark Fieid - Scanning Transmission Electron Microscopy-Aufnahme (HAADF-STEM-Aufnahme) machen zu können.

Die HAADF-STEM-Aufnahme zeigt, dass sich in den Kern/Schale-Partikeln die Partikelgröße der Kernpartikel sowie die Polydispersität der Kern partikel im Wesentlichen nicht geändert haben.

### Beispiel 2

Es wurden als Kern partikel ebenfalls mit Aminopropyltrimethoxysilan oberflächenmodifizierte Siliziumdioxidpartikel eingesetzt, jedoch abweichend von Beispiel 1, mit einer mittleren Partikelgröße von 150 nm.

Zur Herstellung der Suspension der ersten dispersen flüssigen Phase I der späteren Suspoemulsion wurden 1,5 g Zinn(II)chlorid in 64 g Wasser gelöst und anschließend 0,5 g oberflächenmodifizierte Siliziumdioxidpartikel mit einer mittleren Partikelgröße von 150 nm hinzugegeben.

Die zweite kontinuierliche flüssige Phase wurde durch Lösen von 3 g Glissopal® EM23 der BASF AG in 133 g n-Decan hergestellt.

Die erste disperse und die zweite kontinuierliche Phase wurden in einem Ultra-Turrax®-Mischer der Firma IKA®-Werke GmbH & Co. KG (Staufen, Deutschland) voremulgiert. Anschließend wurde die so hergestellte Roh-Suspoemulsion durch Anlegen von Vakuum entgast.

Diese Roh-Suspoemulsion wurde in einem Hochdruckhomogenisator emulgiert, so dass sich eine Tropfengrößenverteilung mit Sauterdurchmesser x_{3,2} von 350 nm einstellte. Anschließend wurde diese Feinemulsion durch Anlegen von Vakuum entgast.

In die Feinemulsion wurden 4,8 g Triethylamin gegeben, so dass sich diese Base in der kontinuierlichen Phase der Suspoemulsion löste. Anschließend wurde auf 130°C erhitzt und diese Temperatur 4 Stunden lang gehalten.

Es wurden Partikel mit einer Kern-Schale-Struktur erhalten, wobei sich Zinndioxid als 5 nm-Partikel auf der Oberfläche der Siliziumdioxid-Kernpartikel ablagerte.

Wie zu Beispiel 1 wurden alle Flüssigkeiten abgetrennt, um eine HAADF-STEM-Aufnahme machen zu können.

Die HAADF-STEM-Aufnahme zeigt, dass sich die Partikelgröße der Kernpartikel sowie die Polydispersität der Kernpartikel durch Aufbringen der Schale nicht wesentlich geändert haben.

### Beispiel 3

Ausgegangen wurde von der Suspension einer ersten, dispersen flüssigen Phase I umfassend mit Aminopropyltrimethoxysilan oberflächenmodifizierte Siliziumdioxidpartikel mit einer mittleren Partikelgröße von 40 nm in Wasser und Zinn(II)chlorid. Die Suspoemulsion umfasste die erste disperse flüssige Phase I in einer zweiten kontinuierlichen flüssigen Phase aus n-Dekan und Glissopal EM23® der BASF AG, einem Tensid aus einem zwitterionischen Molekül auf der Basis von Polyisobuten mit der mittleren Molmasse von ca. 1000 g/mol.

Die erste disperse flüssige Phase wurde hergestellt, indem 0,64 g Zinn(II)chlorid unter Rühren in 63 g Wasser gelöst wurden. Anschließend wurden 0,2 g oberflächenmodifizierte Siliziumdioxidpartikel hinzugegeben.

Die zweite, kontinuierliche flüssige Phase wurde hergestellt, indem 3 g Glissopal EM23® unter Rühren in 133 g n-Dekan gelöst wurden.

Die erste disperse flüssige Phase und die zweite kontinuierliche flüssige Phase wurden in einem Ultra-Turrax®-Mischgerät der Firma IKA®-Werke GmbH & Co. KG (Staufen, Deutschland) voremulgiert. Anschließend wurde die so hergestellte Roh-Suspoemulsion durch Anlegen von Vakuum entgast.

Diese Roh-Suspoemulsion wurde in einem Hochdruckhomogenisator emulgiert, so dass sich eine Tropfengrößenverteilung mit Sauterdurchmesser x_{3,2} von 580 nm einstellte. Anschließend wurde diese Feinemulsion durch Anlegen von Vakuum entgast.

In diese Feinemulsion wurden 2,05 g Trieethylamin hinzugegeben, so dass sich das Triethylamin in der kontinuierlichen Phase der Suspoemulsion löste. Anschließend wurde auf 130°C erhitzt und diese Temperatur 4 Stunden lang gehalten.

Auf diese Weise konnten Kern/Schale-Partikel mit einer Multi-Core/Schalen-Struktur hergestellt werden. Zinnmonoxid lagerte sich als Schale auf der Oberfläche mehrerer Siliziumdioxidkernpartikel ab. Anhand einer EDX-Analyse (energiedispersive Röntgenanalyse) konnte die mittlere Anzahl von Siliziumdioxidkernpartikel pro Multi-Core/Schale-Struktur auf ca. 6 Siliziumdioxidpartikel geschätzt werden.

Von der so erhaltenen Minisuspoemulsion von submikronen Multi-Core/Schale-Partikeln wurden alle Flüssigkeiten abgetrennt, um eine Transmissionselektronenmikroskopieaufnahme (TEM-Aufnahme), genauer eine TEM-Hellfeldabbildung, machen zu können.

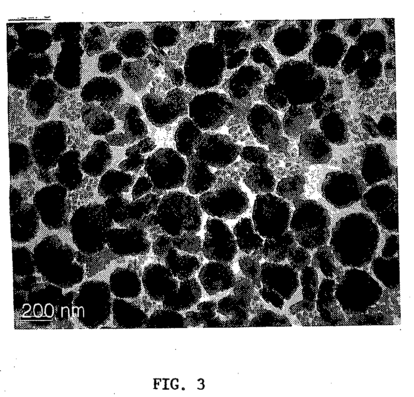

Die TEM-Hellfeldabbildung in Figur 3 zeigt, dass die hergestellten Multi-Core/Schale-Partikeln alle in der gleichen Größenordnung liegen.

In der Zeichnung zeigen im Einzelnen:
- Figur 1: eine HAADF-STEM-Abbildung der nach Ausführungsbeispiel 1 erhaltenen Kern-Schale-Nanopartikel,
- Figur 2: eine HAADF-STEM-Abbildung der nach Ausführungsbeispiel 2 erhaltenen Kern-Schale-Nanopartikel und
- Figur 3: eine TEM-Hellfeldabbildung der nach Ausführungsbeispiel 3 erhaltenen Kern/Schale-Partikel mit einer Multi-Core/Schalen-Struktur.

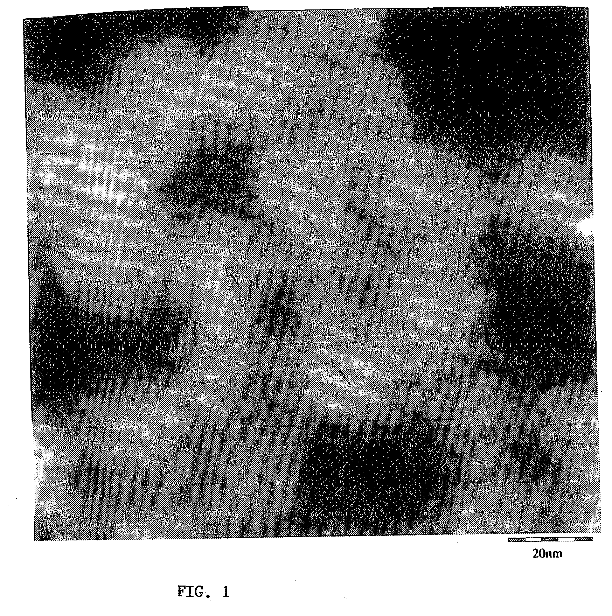

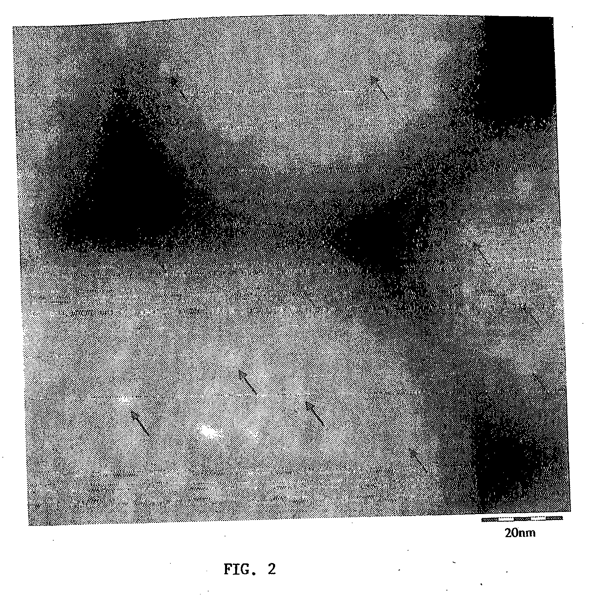

Elemente höherer Ordnung und vergleichsweise dichtere Präparatstellen markieren sich in den TEM-Hellfeldabbildungen (Figur 3) dunkler und in den HAADF-STEM-Abbildungen (Figuren 1 und 2) heller.

Die HAADF-STEM-Aufnahmen in Figur 1 und Figur 2 zeigen, dass Kern-Schale-Nanopartikel mit einem mittleren Partikeldurchmesser in derselben Größenordnung wie die Kernpartikel, von denen in der Herstellung ausgegangen wurde, erhalten wurde.

Die mit den Pfeilen in Figur 1 und Figur 2 hervorgehobenen hellen Bereiche zeigen die Zinndioxid-Partikel, die die Schale der Kern-Schale-Nanopartikel bilden.

Die TEM-Hellfeldabbildung in Figur 3 zeigt eine Vielzahl von Kern/Schale-Partikeln mit einer Multi-Core/Schalen-Struktur, wobei die dunkleren Strukturen in der TEM-Hellfeldabbildung den die Schale bildenden Zinnoxidpartikeln entsprechen.

Die Ausführungsbeispiele verdeutlichen somit, dass die Schalenbildung auf die vorgelegten Kernpartikel sehr gleichmäßig erfolgt und jeder einzelne Kernpartikel eine Schale mit ungefähr derselben Dicke trägt. Das Verfahren ist in einfacher Weise scale-upfähig, weil der mittlere Tropfendurchmesser der dispersen Phase unabhängig von der Menge der Suspoemulsion in derselben Größe einstellbar ist, wobei der mittlere Tropfendurchmesser der dispersen Phase die Größe der Minireaktoren definiert, die gewährleisten, dass die gegebene Polydispersität der sumikronen Ausgangs-Suspension der den Kern bildenden Feststoffe im Produkt, den Kern/Schale-Partikeln im Wesentlichen beibehalten wird.

## Patentansprüche

1. Verfahren zur Herstellung einer Minisuspoemulsion von submikronen Kern/Schale-Partikeln, **dadurch gekennzeichnet, dass**
- von einer Minisuspoemulsion einer ersten, dispersen flüssigen Phase I, in einer zweiten, kontinuierlichen flüssigen Phase II ausgegangen wird, die
- in der ersten, dispersen flüssigen Phase I submikrone Partikel eines den Kern bildenden Feststoffes K sowie
- eine molekular dispers gelöste Vorläufersubstanz VS für die Schale und gegebenenfalls einen Reaktanden R enthält, und dass
- in der ersten, dispersen flüssigen Phase I der Minisuspoemulsion die submikronen Kern/Schale-Partikel KS durch chemische oder physikalische Umwandlung der Vorläufersubstanz VS für die Schale hergestellt werden.

2. Verfahren nach Anspruch 1, **dadurch gekennzeichnet, dass** die Minisuspoemulsion der ersten, dispersen flüssigen Phase I in der zweiten, kontinuierlichen flüssigen Phase II, enthaltend in der ersten, dispersen flüssigen Phase I die submikronen Partikel des den Kern bildenden Feststoffes K sowie die molekular dispers gelöste Vorläufersubstanz VS für die Schale hergestellt wird, indem
- von einer Suspension der submikronen Partikel des den Kern bildenden Feststoffes K in der ersten flüssigen Phase I ausgegangen,
- hierzu die Vorläufersubstanz VS für die Schale zugegeben und molekular dispers gelöst und anschließend
- die zweite flüssige Phase II zugegeben und unter Zuführung von Energie mit der ersten flüssigen Phase I emulgiert wird.

3. Verfahren nach Anspruch 1, **dadurch gekennzeichnet, dass** die Minisuspoemulsion der ersten, dispersen flüssigen Phase I in der zweiten, kontinuierlichen flüssigen Phase II, enthaltend in der ersten, dispersen flüssigen Phase I die submikronen Partikel des den Kern bildenden Feststoffes K sowie die molekular dispers gelöste Vorläufersubstanz VS für die Schale hergestellt wird, indem
- von einer Minisuspoemulsion von submikronen Partikeln eines den Kern bildenden Feststoffes K in der ersten flüssigen Phase I als disperser Phase, in der zweiten flüssigen Phase II als kontinuierlicher Phase ausgegangen wird,
- die Vorläufersubstanz VS für die Schale in einer dritten flüssigen Phase III eingebracht wird, die mit der ersten flüssigen Phase I mischbar ist, jedoch mit der zweiten flüssigen Phase II nicht mischbar ist, und
- aus der dritten flüssigen Phase III, enthaltend die Vorläufersubstanz VS, mit einer vierten flüssigen Phase IV, die mit der zweiten flüssigen Phase II mischbar ist, nicht jedoch mit der ersten und der dritten flüssigen Phase III, unter Energieeintrag eine Emulsion gebildet wird, und
- die Tropfen der ersten flüssigen Phase I und die Tropfen der dritten flüssigen Phase III durch Energieeintrag zur Koaleszenz gebracht werden.

4. Verfahren nach Anspruch 1, **dadurch gekennzeichnet, dass** die Minisuspoemulsion der ersten, dispersen flüssigen Phase I in der zweiten, kontinuierlichen flüssigen Phase II, enthaltend in der ersten, dispersen flüssigen Phase I die submikronen Partikel des den Kern bildenden Feststoffes K, die molekular dispers gelöste Vorläufersubstanz VS für die Schale sowie den Reaktanden R, hergestellt wird, indem
- von einer Minisuspoemulsion von submikronen Partikeln eines den Kern bildenden Feststoffes K sowie einer molekular dispers gelösten Vorläufersubstanz VS für die Schale in der ersten flüssigen Phase I als disperser Phase, in der zweiten flüssigen Phase II als kontinuierlicher Phase ausgegangen wird,
- wonach ein Reaktand R entweder in der zweiten, kontinuierlichen flüssigen Phase II zugegeben wird und in die Tropfen der ersten, dispersen flüssigen Phase I eindiffundiert oder
- in einer weiteren flüssigen Phase V zugegeben wird, die mit der ersten, dispersen flüssigen Phase I mischbar ist, jedoch nicht mit der zweiten, kontinuierlichen flüssigen Phase II
- aus der weiteren flüssigen Phase V, enthaltend den Reaktanden R mit einer zusätzlichen flüssigen Phase VI, unter Energieeintrag eine Emulsion gebildet wird und die Tropfen derselben
- mit den Tropfen der ersten, dispersen flüssigen Phase I, enthaltend den den Kern bildenden Feststoff K sowie die Vorläufersubstanz VS für die Schale, zur erzwungenen Koaleszenz gebracht wird.

5. Verfahren nach Anspruch 1, **dadurch gekennzeichnet, dass** die Minisuspoemulsion der ersten, dispersen flüssigen Phase I in der zweiten, kontinuierlichen flüssigen Phase II, enthaltend in der ersten, dispersen flüssigen Phase I die submikronen Partikel des den Kern bildenden Feststoffes K, die molekular dispers gelöste Vorläufersubstanz VS für die Schale sowie den Reaktanden R, hergestellt wird, indem
- von einer Minisuspoemulsion einer ersten, dispersen flüssigen Phase I, in einer zweiten, kontinuierlichen flüssigen Phase II ausgegangen wird, die in der ersten, dispersen flüssigen Phase I submikrone Partikel eines Feststoffes K enthält,
- ein Reaktand R in einer dritten flüssigen Phase III eingebracht wird, die mit der ersten flüssigen Phase I mischbar ist, jedoch mit der zweiten flüssigen Phase II nicht mischbar ist,
- aus der dritten flüssigen Phase III, enthaltend den Reaktanden R mit einer vierten flüssigen Phase IV unter Energieeintrag eine Emulsion gebildet wird und
- die Tropfen der ersten flüssigen Phase I und die Tropfen der dritten flüssigen Phase III unter Energieeintrag zur Koaleszenz gebracht werden, wonach
- die Vorläufersubstanz VS für die Schale entweder der zweiten, kontinuierlichen flüssigen Phase II zugesetzt wird und in die Tropfen der ersten, dispersen flüssigen Phase I eindiffundiert oder
- in Form einer weiteren Emulsion zugesetzt wird, enthaltend die Vorläufersubstanz VS für die Schale in den Tropfen der dispersen Phase derselben, wobei die disperse Phase der weiteren Emulsion mit der ersten, dispersen flüssigen Phase I mischbar, nicht jedoch mit der kontinuierlichen flüssigen Phase II mischbar ist, und die kontinuierlliche Phase der weiteren Emulsion mit der ersten, kontinuierlichen Phase II mischbar ist, und wonach
- die Tropfen der ersten, dispersen flüssigen Phase I und die Tropfen der flüssigen Phase der weiteren Emulsion zur erzwungenen Koaleszenz gebracht werden.

6. Verfahren nach einem der Ansprüche 1 bis 5, **dadurch gekennzeichnet, dass** die Minisuspoemulsion enthaltend die submikronen Partikel des den Kern bildenden Feststoffes K in der ersten, dispersen flüssigen Phase I, mit der zweiten flüssigen Phase II als kontinuierlicher Phase hergestellt wird, indem
- von einer Miniemulsion ausgegangen wird, enthaltend eine Vorläufersubstanz VK für den den Kern bildenden Feststoff der ersten, dispersen flüssigen Phase I, wobei die zweite flüssige Phase II die kontinuierliche Phase ist, und woraus durch physikalische oder chemische Umwandlung der den Kern bildenden Vorläufersubstanz VK die Minisuspoemulsion des den Kern bildenden Feststoffes K in der ersten, dispersen flüssigen Phase I, mit der zweiten flüssigen Phase II als kontinuierlicher Phase, gebildet wird.

7. Verfahren nach Anspruch 6, **dadurch gekennzeichnet, dass** die Minisuspoemulsion des den Kern bildenden Feststoffes K in der ersten flüssigen Phase I als disperser Phase, mit der zweiten flüssigen Phase II als kontinuierlicher Phase gebildet wird, indem
- von einer Miniemulsion ausgegangen wird, enthaltend eine Vorläufersubstanz VK für den den Kern bildenden Feststoff in der ersten, dispersen flüssigen Phase I, wobei die zweite flüssige Phase II die kontinuierliche Phase ist,
- wonach ein Reaktand R entweder der zweiten, kontinuierlichen flüssigen Phase II zugesetzt wird und in die Tropfen der ersten flüssigen Phase I eindiffundiert oder
- in einer dritten flüssigen Phase III zugesetzt wird, die mit der ersten flüssigen Phase I mischbar ist, jedoch mit der zweiten flüssigen Phase II nicht mischbar ist, und
- aus der dritten flüssigen Phase III, enthaltend den Reaktanden R mit einer vierten flüssigen Phase IV, die mit der zweiten flüssigen Phase II mischbar ist, nicht jedoch mit der ersten und der dritten flüssigen Phase, unter Energieeintrag eine Emulsion gebildet und deren Tropfen mit den Tropfen der ersten, dispersen flüssigen Phase I, enthaltend die Vorläufersubstanz VK für den den Kern bildenden Feststoff zur erzwungenen Koaleszenz gebracht werden, und wonach
- die Vorläufersubstanz VK für den den Kern bildenden Feststoff K mit dem Reaktanden R zur chemischen Umsetzung gebracht wird.

8. Verfahren nach einem der Ansprüche 1 bis 7, **dadurch gekennzeichnet, dass** aus der Minisuspoemulsion der submikronen Kern/Schale-Partikel durch Abziehen der ersten, dispersen flüssigen Phase I oder der zweiten kontinuierlichen flüssigen Phase II eine Suspension von submikronen Kern/Schale-Partikeln in der jeweils anderen flüssigen Phase erhalten wird.

9. Verfahren nach einem der Ansprüche 1 bis 8, **dadurch gekennzeichnet, dass** 2 oder mehrere, insbesondere 2 bis 1000 den Kern bildende Partikel von einer Schale umhüllt werden.

10. Verfahren nach einem der Ansprüche 1 bis 9, **dadurch gekennzeichnet, dass** auf die den Kern bildenden Partikel 2 bis 10, bevorzugt 2 bis 3, Schalen übereinander aufgebracht werden.

11. Verfahren nach einem der Ansprüche 1 bis 10, **dadurch gekennzeichnet, dass** eine Minisuspoemulsion von submikronen Kern/Schale-Partikeln hergestellt wird, bei denen die Schale den Kern nicht vollständig umschließt und dass die submikronen Kern/Schale-Partikel einem nachgeschalteten Verfahrensschritt unterworfen werden, indem der Kern vollständig oder teilweise, insbesondere durch Verdampfen, Lösen oder Ätzen, unter Erhalt einer Hohlstruktur entfernt wird und auf dieselbe bevorzugt eine oder mehrere weitere Schalen aufgebracht werden können.

12. Verfahren nach einem der Ansprüche 1 bis 3 oder 6 bis 11, **dadurch gekennzeichnet, dass** die physikalische Umwandlung durch Veränderung eines oder mehrerer Verfahrensparameter, insbesondere der Temperatur und/oder des Druckes oder durch Zugabe eines Lösungsmittels oder eines Salzes analog der Zugabe des Reaktanden R erfolgt.

13. Verfahren nach einem der Ansprüche 1 bis 12, **dadurch gekennzeichnet, dass** der den Kern bildende Feststoff K oder der die Schale bildende Feststoff S eine Substanz oder eine Mischung von Substanzen, ausgewählt aus der nachfolgenden Aufzählung, ist: Metalle, Oxide, Halbleiter, Ruß, Metallsalze, Schwefel, Schwefelverbindungen, Siliziumverbindungen, Polymere, anorganische oder organische Pigmente oder feste anorganische oder organische Wirkstoffe für Kosmetika, Pflanzenschutz-, Tierernährungs- oder Nahrungsergänzungsmittel.

14. Verfahren nach einem der Ansprüche 1 bis 13, **dadurch gekennzeichnet, dass** als molekulardisperse Vorläufersubstanz VS des die Schale bildenden Feststoffes S oder als Vorläufersubstanz VK des den Kern bildenden Feststoffes K ein oder mehrere organische oder anorganische Salze, insbesondere Zinnsalze, Zinksalze, Cersalze, Eisensalze, Zirkoniumsalze, Bismutsalze oder Kupfersalze oder anorganische Metallverbindungen, insbesondere des Titans, Monomere und/oder ein oder mehrere Siliziumverbindungen eingesetzt werden.

15. Verwendung der in einem Verfahren nach einem der Ansprüche 1 bis 14 hergestellten Minisuspoemulsion von submikronen Kern/Schale-Partikeln als Katalysator, als Wirkstoff-Formulierung, insbesondere in Kosmetika, Pflanzenschutz-, Tierernährungs- oder Nahrungsergänzungsmitteln, als Pigment, in der Elektronik, in optischen Anwendungen oder in Polymeren.

## Claims

1. A process for the preparation of a minisuspoemulsion of submicron core/shell particles, wherein
- the starting material is a minisuspoemulsion of a first disperse liquid phase I in a second continuous liquid phase II, which
- comprises, in the first disperse liquid phase I, submicron particles of a solid C forming the core, and
- a precursor substance PS for the shell dissolved in molecularly disperse form and optionally a reactant R, and wherein
- the submicron core/shell particles CS are prepared in the first disperse liquid phase I of the minisuspoemulsion by chemical or physical conversion of the precursor substance PS for the shell.

2. The process according to claim 1, wherein the minisuspoemulsion of the first disperse liquid phase I in the second continuous liquid phase II, comprising, in the first disperse liquid phase I, the submicron particles of the solid C forming the core and also the precursor substance PS, for the shell dissolved in molecularly disperse form, is prepared by
- starting from a suspension of the submicron particles of the solid C forming the core in the first liquid phase I,
- adding the precursor substance PS for the shell thereto and dissolving in molecularly disperse form, and subsequently
- adding the second liquid phase II and emulsifying with the first liquid phase I while providing energy.

3. The process according to claim 1, wherein the minisuspoemulsion of the first disperse liquid phase I in the second continuous liquid phase II, comprising, in the first disperse liquid phase I, the submicron particles of the solid C forming the core and also the precursor substance PS for the shell dissolved in molecularly disperse form, is prepared by
- starting from a minisuspoemulsion of submicron particles of a solid C forming the core in the first liquid phase I, as disperse phase, in the second liquid phase II, as continuous phase,
- introducing the precursor substance PS for the shell into a third liquid phase III which is miscible with the first liquid phase I but is immiscible with the second liquid phase II, and
- forming an emulsion from the third liquid phase III, comprising the precursor substance PS, with a fourth liquid phase IV, which is miscible with the second liquid phase II but immiscible with the first and the third liquid phase III, while supplying energy, and
- bringing the drops of the first liquid phase I and the drops of the third liquid phase III to coalescence by supplying energy.

4. The process according to claim 1, wherein the minisuspoemulsion of the first disperse liquid phase I in the second continuous liquid phase II, comprising, in the first disperse liquid phase I, the submicron particles of the solid C forming the core, the precursor substance PS for the shell dissolved in molecularly disperse form and also the reactant R, is prepared by
- starting from a minisuspoemulsion of submicron particles of a solid C forming the core and also a precursor substance PS for the shell dissolved in molecularly disperse form in the first liquid phase I, as disperse phase, in the second liquid phase II, as continuous phase,
- after which a reactant R either is added to the second continuous liquid phase II and diffuses into the drops of the first disperse liquid phase I or
- is added to an additional liquid phase V which is miscible with the first disperse liquid phase I but immiscible with the second continuous liquid phase II,
- an emulsion is formed from the additional liquid phase V, comprising the reactant R, with an additional liquid phase VI, while supplying energy, and the drops of the same
- are brought to forced coalescence with the drops of the first disperse liquid phase I comprising the solid C forming the core and also the precursor substance PS for the shell.

5. The process according to claim 1, wherein the minisuspoemulsion of the first disperse liquid phase I in the second continuous liquid phase II, comprising, in the first disperse liquid phase I, the submicron particles of the solid C forming the core, the precursor substance PS for the shell dissolved in molecularly disperse form and also the reactant R, is prepared by
- starting from a minisuspoemulsion of a first disperse liquid phase I in a second continuous liquid phase II comprising, in the first disperse liquid phase I, submicron particles of a solid C,
- introducing a reactant R into a third liquid phase III which is miscible with the first liquid phase I but immiscible with the second liquid phase II,
- forming an emulsion from the third liquid phase III, comprising the reactant R, with a fourth liquid phase IV, while supplying energy, and
- bringing the drops of the first liquid phase I and the drops of the third liquid phase III to coalescence while supplying energy, after which
- the precursor substance PS for the shell either is added to the second continuous liquid phase II and diffuses into the drops of the first disperse liquid phase I or
- is added in the form of an additional emulsion comprising the precursor substance PS for the shell in the drops of the disperse phase of the same, the disperse phase of the additional emulsion being miscible with the first disperse liquid phase I but immiscible with the continuous liquid phase II and the continuous phase of the additional emulsion being miscible with the first continuous phase II, and after which
- the drops of the first disperse liquid phase I and the drops of the liquid phase of the additional emulsion are brought to forced coalescence.

6. The process according to any of claims 1 to 5, wherein the minisuspoemulsion comprising the submicron particles of the solid C forming the core in the first disperse liquid phase I, with the second liquid phase II as continuous phase, is prepared by
- starting from a miniemulsion comprising a precursor substance PC for the solid forming the core of the first disperse liquid phase I, the second liquid phase II being the continuous phase, and by forming therefrom the minisuspoemulsion of the solid C forming the core in the first disperse liquid phase I, with the second liquid phase II as continuous phase, by physical or chemical conversion of the precursor substance PC forming the core.

7. The process according to claim 6, wherein the minisuspoemulsion of the solid C forming the core in the first liquid phase I as disperse phase, with the second liquid phase II as continuous phase, is formed by
- starting from a miniemulsion comprising a precursor substance PC for the solid forming the core in the first disperse liquid phase I, the second liquid phase II being the continuous phase,
- after which the reactant R either is added to the second continuous liquid phase II and diffuses into the drops of the first liquid phase I or
- is added to a third liquid phase III which is miscible with the first liquid phase I but is immiscible with the second liquid phase II, and
- an emulsion is formed from the third liquid phase III, comprising the reactant R, with a fourth liquid phase IV which is miscible with the second liquid phase II but immiscible with the first and the third liquid phase, while supplying energy, and the drops thereof are brought to forced coalescence with the drops of the first disperse liquid phase I comprising the precursor substance PC for the solid forming the core, and after which
- the precursor substance PC for the solid C forming the core is reacted chemically with the reactant R.

8. The process according to any of claims 1 to 7, wherein, starting from the minisuspoemulsion of the submicron core/shell particles, by drawing off the first disperse liquid phase I or the second continuous liquid phase II, a suspension of submicron core/shell particles in the other liquid phase each time is obtained.

9. The process according to any of claims 1 to 8, wherein 2 or more, in particular from 2 to 1000, of the particles forming the core are enveloped by one shell.

10. The process according to any of claims 1 to 9, wherein 2 to 10, preferably 2 to 3, shells are applied one above the other to the particles forming the core.

11. The process according to any of claims 1 to 10, wherein a minisuspoemulsion of submicron core/shell particles is prepared in which the shell does not completely enclose the core and wherein the submicron core/shell particles are subjected to a downstream processing stage in which the core is completely or partially removed, in particular by evaporation, dissolution or corrosion, while preserving a hollow structure, and one or more additional shells can preferably be applied to the same.

12. The process according to any of claims 1 to 3 or 6 to 11, wherein the physical conversion is carried out by change in one or more processing parameters, in particular the temperature and/or the pressure, or by addition of a solvent or a salt analogously to the addition of the reactant R.

13. The process according to any of claims 1 to 12, wherein the solid C forming the core or the solid S forming the shell is a substance or a mixture of substances chosen from the following list: metals, oxides, semiconductors, carbon blacks, metal salts, sulfur, sulfur compounds, silicon compounds, polymers, inorganic or organic pigments or solid inorganic or organic active substances for cosmetics, pesticides, animal nutritional agents or food supplements.

14. The process according to any of claims 1 to 13, wherein use is made, as molecularly disperse precursor substance PS of the solid S forming the shell or as precursor substance PC of the solid C forming the core, of one or more organic or inorganic salts, in particular tin salts, zinc salts, cerium salts, iron salts, zirconium salts, bismuth salts or copper salts, or inorganic metal compounds, in particular of titanium, monomers and/or one or more silicon compounds.

15. The use of the minisuspoemulsion of submicron core/shell particles prepared in a process according to any of claims 1 to 14 as catalyst, as active substance formulation, in particular in cosmetics, pesticides, animal nutritional agents or food supplements, as pigment, in electronics, in optical applications or in polymers.

## Revendications

1. Procédé de fabrication d'une mini-suspoémulsion de particules coeur/coque submicroniques, **caractérisé en ce que**
- on part d'une mini-suspoémulsion d'une première phase liquide dispersée I dans une deuxième phase liquide continue II, qui contient
- dans la première phase liquide dispersée I des particules submicroniques d'un solide K formant le noyau, ainsi que
- une substance précurseur VS, dissoute en dispersion moléculaire, pour la coque, et éventuellement un réactif R, et que
- dans la première phase liquide dispersée I de la mini-suspoémulsion, les particules coeur/coque submicroniques KS sont fabriquées par conversion chimique ou physique de la substance précurseur VS pour la coque.

2. Procédé selon la revendication 1, **caractérisé en ce qu'**on fabrique la mini-suspoémulsion de la première phase liquide dispersée I dans la deuxième phase liquide continue II, contenant dans la première phase liquide dispersée I les particules submicroniques du solide K formant le coeur, ainsi que la substance précurseur dissoute en dispersion moléculaire VS pour la coque, par le fait que
- on part d'une suspension des particules submicroniques du solide formant le coeur K dans la première phase liquide I,
- on lui ajoute la substance précurseur VS pour la coque, et on la dissout en dispersion moléculaire, puis
- on ajoute la deuxième phase liquide II et on l'émulsionne, avec apport d'énergie, avec la première phase liquide I.

3. Procédé selon la revendication 1, **caractérisé en ce qu'**on fabrique la mini-suspoémulsion de la première phase liquide dispersée I dans la deuxième phase liquide continue II, contenant dans la première phase liquide dispersée I les particules submicroniques du solide K formant le coeur, ainsi que la substance précurseur dissoute en dispersion moléculaire VS pour la coque, par le fait que
- on part d'une mini-suspoémulsion de microparticules submicroniques d'un solide K formant le coeur dans la première phase liquide I en tant que phase dispersée, dans la deuxième phase liquide II en tant que phase continue,
- on introduit la substance précurseur VS pour la coque dans une troisième phase liquide III, qui est miscible à la première phase liquide I mais n'est pas miscible à la deuxième phase liquide II,
- à partir de la troisième phase liquide III, qui contient la substance précurseur VS, on forme une émulsion, avec apport d'énergie, avec une quatrième phase liquide IV, qui est miscible à la deuxième phase liquide II mais non pas à la première et à la troisième phase liquide III, et
- on porte à coalescence avec apport d'énergie les gouttes de la première phase liquide I et les gouttes de la troisième phase liquide III.

4. Procédé selon la revendication 1, **caractérisé en ce qu'**on fabrique la mini-suspoémulsion de la première phase liquide dispersée I dans la deuxième phase liquide continue II, contenant dans la première phase liquide dispersée I les particules submicroniques du solide K formant le coeur, la substance précurseur dissoute en dispersion moléculaire VS pour la coque, ainsi que le réactif R, par le fait que
- on part d'une mini-suspoémulsion de particules submicroniques d'un solide K formant le coeur, ainsi que d'une substance précurseur VS, dissoute en dispersion moléculaire, pour la coque, dans la première phase liquide I en tant que phase dispersée, dans la deuxième phase liquide II en tant que phase continue,
- ce après quoi ou bien on ajoute un réactif R à la deuxième phase liquide continue II, et on l'introduit par diffusion dans les gouttes de la première phase liquide dispersée I, ou bien
- on l'ajoute à une phase liquide supplémentaire V, qui est miscible à la première phase liquide dispersée I mais non pas à la deuxième phase liquide continue II,
- à partir de la phase liquide supplémentaire V, contenant le réactif R, on forme une émulsion avec apport d'énergie avec une phase liquide supplémentaire VI, et on porte à coalescence forcée les gouttes de cette dernière,
- avec les gouttes de la première phase liquide dispersée I, contenant le solide K formant le coeur, ainsi que la substance précurseur VS pour la coque.

5. Procédé selon la revendication 1, **caractérisé en ce qu'**on fabrique la mini-suspoémulsion de la première phase liquide dispersée I dans la deuxième phase liquide continue II, contenant dans la première phase liquide dispersée I les particules submicroniques du solide K formant le coeur, la substance précurseur VS, dissoute en dispersion moléculaire, pour la coque, ainsi que le réactif R, par le fait que
- on part d'une mini-suspoémulsion d'une première phase liquide dispersée I dans une deuxième phase liquide continue II qui dans la première phase liquide dispersée I contient des particules submicroniques d'un solide K,
- on introduit un réactif R dans une troisième phase liquide III, qui est miscible à la première phase liquide I, mais qui n'est pas miscible à la deuxième phase liquide II,
- à partir de la troisième phase liquide III, contenant le réactif R, on forme avec une quatrième phase liquide IV une émulsion avec apport d'énergie, et
- on porte à coalescence avec apport d'énergie les gouttes de la première phase liquide I et les gouttes de la troisième phase liquide III, ce après quoi
- ou bien on ajoute la substance précurseur VS pour la coque à la deuxième phase liquide continue II, et on l'introduit par diffusion dans les gouttes de la première phase liquide dispersée I, ou bien
- on l'ajoute sous forme d'une émulsion supplémentaire, contenant la substance précurseur VS pour la coque dans les gouttes de sa phase dispersée, la phase dispersée de l'émulsion supplémentaire étant miscible à la première phase liquide dispersée I mais n'étant pas miscible à la phase liquide continue II, et la phase continue de l'émulsion supplémentaire étant miscible à la première phase continue II, ce après quoi
- on porte à coalescence forcée les gouttes de la première phase liquide dispersée I et les gouttes de la phase liquide de l'émulsion supplémentaire.

6. Procédé selon l'une des revendications 1 à 5, **caractérisé en ce qu'**on fabrique la mini-suspoémulsion contenant les particules submicroniques du solide K formant le coeur dans la première phase liquide dispersée I, avec la deuxième phase liquide II en tant que phase continue, par le fait que
- on part d'une mini-émulsion, contenant une substance précurseur VK pour le solide formant le coeur de la première phase liquide dispersée I, la deuxième phase liquide II étant la phase continue, et à partir de laquelle, par conversion physique ou chimique de la substance précurseur VK formant le coeur, on forme la mini-suspoémulsion du solide K formant le coeur dans la première phase liquide dispersée I, avec la deuxième phase liquide II en tant que phase continue.

7. Procédé selon la revendication 6, **caractérisé en ce qu'**on forme la mini-suspoémulsion du solide K formant le coeur dans la première phase liquide I en tant que phase dispersée, avec la deuxième phase liquide II en tant que phase continue, par le fait que
- on part d'une mini-émulsion contenant une substance précurseur VK pour le solide formant le coeur dans la première phase liquide dispersée I, la deuxième phase liquide II étant la phase continue,
- puis ou bien on ajoute un réactif R à la deuxième phase liquide continue II et on l'introduit par diffusion dans les gouttes de la première phase liquide I, ou bien
- on l'ajoute à une troisième phase liquide III, qui est miscible à la première phase liquide I mais n'est pas miscible à la deuxième phase liquide II, et
- à partir de la troisième phase liquide III, contenant le réactif R, on forme avec apport d'énergie une émulsion avec une quatrième phase liquide IV qui est miscible à la deuxième phase liquide II mais non pas à la première et à la troisième phase liquide, et on porte à coalescence forcée ses gouttes avec les gouttes de la première phase liquide dispersée I, contenant la substance précurseur VK pour le solide formant le coeur, ce après quoi,
- on soumet à une réaction chimique la substance précurseur VK pour le solide K formant le coeur, avec le réactif R.

8. Procédé selon l'une des revendications 1 à 7, **caractérisé en ce qu'**on obtient, à partir de la mini-suspoémulsion des particules coeur/coque submicroniques, par extraction de la première phase liquide continue I ou de la deuxième phase liquide continue II, une suspension de particules coeur/coque submicroniques dans l'autre phase liquide correspondante.

9. Procédé selon l'une des revendications 1 à 8, **caractérisé en ce que** 2 ou plus, en particulier 2 à 1000 particules formant le coeur sont entourées d'une coque.

10. Procédé selon l'une des revendications 1 à 9, **caractérisé en ce qu'**on applique les unes sur les autres 2 à 10, de préférence 2 à 3, coques sur les particules formant le coeur.

11. Procédé selon l'une des revendications 1 à 10, **caractérisé en ce qu'**on fabrique une mini-suspoémulsion de particules coeur/coque submicroniques, dans lesquelles la coque n'entoure pas complètement le coeur, et que les particules coeur/coque submicroniques sont soumises à une étape de procédé subséquente, dans laquelle le coeur est éliminé en totalité ou en partie, en particulier par évaporation, dissolution ou attaque chimique, avec obtention d'une structure creuse, une ou plusieurs coques supplémentaires pouvant être appliquées de préférence sur cette dernière.

12. Procédé selon l'une des revendications 1 à 3 ou 6 à 11, **caractérisé en ce que** la conversion physique a lieu par modification d'un ou plusieurs paramètres du procédé, en particulier la température et/ou la pression, ou par addition d'un solvant ou d'un sel, d'une manière analogue à l'addition du réactif R.

13. Procédé selon l'une des revendications 1 à 12, **caractérisé en ce que** le solide K formant le coeur ou le solide S formant la coque est une substance ou un mélange de substances choisies parmi la liste suivante :
les métaux, les oxydes, les semi-conducteurs, le noir de carbone, les sels métalliques, le soufre, les composés du soufre, les composés du silicium, les polymères, les pigments inorganiques ou organiques, ou
les matières actives inorganiques ou organiques solides pour produits cosmétiques, produits phytosanitaires, produits pour l'alimentation animale ou pour compléments alimentaires.

14. Procédé selon l'une des revendications 1 à 13, **caractérisé en ce qu'**on utilise en tant que substance précurseur VS en dispersion moléculaire du solide S formant la coque ou en tant que substance précurseur VK du solide K formant le coeur un ou plusieurs sels organiques ou inorganiques, en particulier des sels d'étain, des sels de zinc, des sels de cérium, des sels de fer, des sels de zirconium, des sels de bismuth ou des sels de cuivre, ou des composés métalliques inorganiques, en particulier du titane, des monomères et/ou un ou plusieurs composés du silicium.

15. Utilisation de la mini-suspoémulsion de particules coeur/coque submicroniques fabriquée dans un procédé selon l'une des revendications 1 à 14 en tant que catalyseur, en tant que formulation de matière active, en particulier dans les produits cosmétiques, les produits phytosanitaires, les produits pour l'alimentation animale ou pour compléments alimentaires, en tant que pigments, en électronique, dans les applications optiques ou dans les polymères.
